(19) Europäisches Patentamt
European Patent Office
Office européen des brevets

(11) Veröffentlichungsnummer: **0 158 789**
**A1**

(12) # EUROPÄISCHE PATENTANMELDUNG

(21) Anmeldenummer: 85101912.5

(22) Anmeldetag: 21.02.85

(51) Int. Cl.[4]: **C 07 D 295/08**
C 07 D 307/52, C 07 D 285/10
A 61 K 31/445, A 61 K 31/425
A 61 K 31/34

(30) Priorität: 07.03.84 DE 3408327

(43) Veröffentlichungstag der Anmeldung:
23.10.85 Patentblatt 85/43

(84) Benannte Vertragsstaaten:
AT BE CH DE FR GB IT LI LU NL SE

(71) Anmelder: LUDWIG HEUMANN & CO GMBH
Heideloffstrasse 18-28 Postfach 2260
D-8500 Nürnberg(DE)

(72) Erfinder: Postius, Stefan, Dr.
Nunnenbeckstrasse 24
D-8500 Nürnberg 20(DE)

(72) Erfinder: Herter, Rolf, Dr. Dipl.-Chem.
Bayernstrasse 42
D-8540 Schwabach(DE)

(72) Erfinder: Mörsdorf, Peter, Dr. Dipl.-Chem.
Untere Bahnhofstrasse 16
D-8501 Cadolzburg(DE)

(72) Erfinder: Schickaneder, Helmut, Dr. Dipl.-Chem.
Moosäcker 25
D-8501 Eckental(DE)

(72) Erfinder: Szelenyi, Istvan, Dr.
Brahmsstrasse 16
D-8501 Schwaig(DE)

(72) Erfinder: Ahrens, Kurt Henning, Dr.
Praterstrasse 9
D-8500 Nürnberg(DE)

(74) Vertreter: Kraus, Walter, Dr. et al,
Patentanwälte Kraus Weisert & Partner
Thomas-Wimmer-Ring 15
D-8000 München 22(DE)

(54) **Diaminderivate, Verfahren zu ihrer Herstellung und diese Verbindungen enthaltende Arzneimittel.**

(57) Es werden Diaminderivate der allgemeinen Formel I

$R^1R^2N-(CH_2)_m-Q-A-NH-R-NH-B-Q'-(CH_2)_nNR^3R^4$

beschrieben, die eine hohe selektive Wirkung auf Histamin-$H_2$-Rezeptoren besitzen und somit als Antiulcusmittel geeignet sind. Es werden auch Verfahren zur Herstellung dieser Verbindungen und diese Verbindungen enthaltende Arzneimittel beschrieben.

EP 0 158 789 A1

# Diaminderivate, Verfahren zu ihrer Herstellung und diese Verbindungen enthaltende Arzneimittel

Die Erfindung betrifft neue Diaminderivate mit einer hohen selektiven Wirkung auf Histamin-$H_2$-Rezeptoren, Verfahren zu ihrer Herstellung und Arzneimittel, die diese Verbindungen enthalten, sowie schließlich die Verwendung dieser Verbindungen in der Therapie.

Als Antiulcusmittel haben Cimetidin und Ranitidin bereits Eingang in die Therapie gefunden. Die Halbwertszeiten von Cimetidin und Ranitidin sind jedoch verhältnismäßig kurz. Dadurch wird es nötig, mehrfach täglich Tabletten mit Dosiseinheiten von 160 bis 300 mg in einer therapeutisch festgelegten Form zu verabreichen. Es besteht somit ein Bedarf an Antiulcusmitteln, die länger wirken und/oder wirksamer sind als Cimetidin und Ranitidin.

Verbindungen dieser Art zeigen aufgrund ihrer spezifischen $H_2$-antagonistischen Aktivität eine Hemmung der Magensäuresekretion, wenn diese durch Histaminagonisten stimuliert wird [Ash und Schild, "Brit. J. Pharmacol. Chemother.", 27, 427 (1966) und Black et al., "Nature", 236, 385 (1971)]. Die pharmakologische Aktivität dieser Verbindungen, wie sie genauer weiter unten beschrieben werden, kann nach einer modifizierten Methode gemäß der DE-OS 27 34 070 beim perfundierten Rattenmagen gezeigt werden oder durch die Ermittlung der $pA_2$-Werte in vitro am Meerschweinchenvorhof (vgl. Ariens, Molecular Pharmacology, Band 1, Academic Press, New York, 1964) nachgewiesen werden. Weiterhin kann die $H_2$-antagonistische Wirkung an wachen Heidenhain-Pouch-Hunden nach der Methode von Black et al., "Nature", 236, 385 (1971) und an wachen Fistelkatzen gezeigt werden. Solche Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden.

Nachdem Hemmstoffe für Histamin-$H_2$-Rezeptoren sowohl bezüglich der basalen Magensäuresekretion als auch der durch Gastrin, Histamin, Metacholin oder Nahrung induzierten Magensäuresekretion Hemmwirkung besitzen, können sie für die Behandlung von peptischen Ulcera, die durch übermäßige Sekretion von Magensäure verursacht sind, und bei der Therapie hyperacider Gastritis verwendet werden.

Der Erfindung liegt die Aufgabe zugrunde, neue Hemmstoffe für Histamin-$H_2$-Rezeptoren mit verbesserter und/oder länger anhaltender Wirksamkeit zur Verfügung zu stellen.

Diese Aufgabe wird durch die Erfindung gelöst.

Gegenstand der Erfindung sind neue Diaminderivate, wie sie in den Ansprüchen gekennzeichnet sind.

In der allgemeinen Formel I

$$R^1R^2N-(CH_2)_m-Q-A-NH-R-NH-B-Q'-(CH_2)_nNR^3R^4 \qquad (I)$$

haben m und n unabhängig voneinander den Wert 0 oder 1. $R^1$, $R^2$, $R^3$ und $R^4$ stehen unabhängig voneinander jeweils für Wasserstoff, $C_{1-10}$-Alkyl, insbesondere $C_{1-3}$-Alkyl, wie Methyl oder Ethyl, $C_{5-6}$-Cycloalkyl, d.h. Cyclopentyl oder Cyclohexyl, Amino, Niedrigalkylamino, insbesondere Propyl-, Ethyl- oder Methylamino, Diniedrigalkylamino, insbesondere Dipropyl-, Diethyl- oder Dimethylalkylamino. $R^1$ und $R^2$ einerseits und/oder $R^3$ und $R^4$ andererseits können weiterhin zusammen mit dem Stickstoffatom, an das sie jeweils gebunden sind, einen 5- bis 8gliedrigen alicyclischen heterocyclischen Ring bilden. Dieser kann substituiert oder unsubstituiert sein. Bevorzugte Beispiele für solche Ringe sind der Pyrrolidin-, Methylpyrrolidin-, Morpholin-, Thiomorpholin-, Piperidin-, Methylpiperidin-, N-Methylpiperazin-, Homopiperidin-, Heptamethylenimino- und Octamethyleniminoring. Bevorzugt werden der Pyrrolidin- und Piperidinring. Ungeachtet des Wertes für m und n können $R^3$ und $R^4$ miteinander die Gruppierung $(NH_2)_2C=$ bilden. Im Falle, daß m den Wert 0 hat, können $R^1$ und $R^2$ miteinander die Gruppierung $(NH_2)_2C=$ bilden.

In der allgemeinen Formel I bedeuten weiterhin Q und Q', die gleich oder verschieden sein können, einen Furan-, Thiophen-, Thiazol- oder Benzolring, wobei der Benzolring bevorzugt wird. Der Benzolring ist vorzugsweise in 1,3- oder 1,4-Position in den Rest des Moleküls eingefügt. Der Furanring ist vorzugsweise in 2,5-Position in den Rest des Moleküls eingefügt. Der Thiophenring ist vorzugsweise in 2,4- oder 2,5-Position in den Rest des Moleküls eingefügt. Der Thiazolring ist vorzugsweise in 2,4-Position in den Rest des Moleküls eingefügt. A und B stehen unabhängig voneinander für die Gruppierung $-O-(CH_2)_q-$, $-O-CH_2-CHOH-CH_2-$ oder $-CH_2-X-CH_2-Y-(CH_2)_p-$, vorzugsweise für die Gruppierung $-O-CH_2-CHOH-CH_2-$. In der erstgenannten Gruppierung hat q den Wert 2, 3 oder 4, vorzugsweise 2 oder 3. In der letztgenannten Gruppierung ist X ein Schwefel- oder Sauerstoffatom oder die Gruppe -CHOH, vorzugsweise ein Sauerstoffatom. p hat den Wert 1 oder 2, und Y stellt eine Einfachbindung oder die Gruppe -CHOH dar. R steht für das Ringsystem

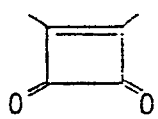

oder

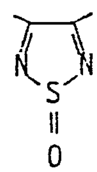

Bei einer bevorzugten Gruppe von Verbindungen steht in der allgemeinen Formel I Q für einen Benzolring, dessen Einfügung in den Rest des Moleküls durch Bindung in 1- und 3- oder 1- und 4-Stellung, vorzugsweise in 1- und 3-Stellung, erfolgt ist. In diesem Falle haben m und n jeweils den Wert 1. $R^1$ und $R^3$, die in diesem Fall gleich sind, bedeuten jeweils vorzugsweise ein Wasserstoffatom oder lineares $C_{1-10}$-Alkyl, insbesondere $C_{1-3}$-Alkyl, wie zum Beispiel Methyl, Ethyl, Propyl oder Isopropyl. $R^2$ und $R^4$, die in diesem Falle gleich sind, bedeuten $C_{5-6}$-Cycloalkyl, wie Cyclopentyl oder Cyclohexyl, vorzugsweise Cyclohexyl. Stehen $R^1$ und $R^3$ für lineares $C_{1-10}$-Alkyl, insbesondere $C_{1-3}$-Alkyl, wie zum Beispiel Methyl, Ethyl oder Propyl, dann bedeuten $R^2$ und $R^4$ vorzugsweise ebenfalls lineares $C_{1-10}$-Alkyl, insbesondere $C_{1-3}$-Alkyl, wie oben definiert.

Eine weitere bevorzugte Gruppe von erfindungsgemäßen Verbindungen ist dadurch gekennzeichnet, daß $R^1$ und $R^2$ und/oder $R^3$ und $R^4$ zusammen mit dem Stickstoffatom, an das sie gebunden sind, einen substituierten oder unsubstituierten 5- bis 8gliedrigen alicyclischen heterocyclischen Ring, wie zum Beispiel einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring, insbesondere einen Pyrrolidinring oder Piperidinring, bilden. In diesem Fall bedeuten in der allgemeinen Formel I weiterhin A die Gruppierung $-O-(CH_2)_q-$, $-O-CH_2-CHOH-CH_2-$, $-CH_2-O-CH_2-CHOH-CH_2-$, $-CH_2-CHOH-CH_2-$ oder $-CH_2-CHOH-CH_2CH_2-$, R das Ringsystem

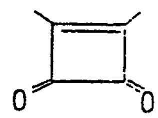 oder 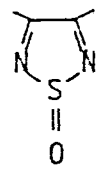

während B für eine Gruppierung, wie unter A beschrieben, steht und Q und Q' einen in 1,3-Position eingefügten Benzolring bedeuten.

Besonders bevorzugt werden Verbindungen, bei denen m und n jeweils den Wert 1 haben, $R^1$ und $R^2$ sowie $R^3$ und $R^4$ zusammen mit dem jeweiligen Stickstoffatom einen Pyrrolidin- oder Piperidinring bilden, Q und Q' jeweils einen Benzolring darstellen, dessen Einfügung in den Rest des Moleküls durch Bindungen in 1- und 3-Position erfolgt, A die Gruppierung $-O-(CH_2)_3-$ oder A und B die Gruppierung $-O-CH_2-CHOH-CH_2-$ oder A die Gruppierung $-O-(CH_2)_3-$ und B die Gruppierung $-O-CH_2-CHOH-CH_2-$ bedeuten und R eines der oben genannten Ringsysteme darstellt.

Bei einer weiteren bevorzugten Gruppe der oben beschriebenen Art bedeutet A die Gruppierung $-O-(CH_2)_q-$, $-O-CH_2-CHOH-CH_2-$, $-CH_2-O-CH_2-CHOH-CH_2-$, $-CH_2-CHOH-CH_2-$ oder $-CH_2-CHOH-CH_2CH_2-$ und R eines der oben erwähnten Ringsysteme. B steht für die Gruppierung $-CH_2-X-CH_2-Y-(CH_2)_p-$, wobei X ein Schwefelatom oder die Gruppe -CHOH darstellt. Y ist in diesem Falle eine Einfachbindung oder die Gruppe -CHOH. p hat den Wert 1 oder 2. Q' steht für einen in 2,5-Stellung in den Rest des Moleküls eingefügten Furanring oder einen in 2,5- bzw. 2,4-Stellung in den Rest des Moleküls eingefügten Thiophenring.

0158789

Ganz besonders bevorzugt werden Verbindungen, bei denen m und n jeweils den Wert 1 haben und $R^1$ und $R^2$ zusammen mit dem Stickstoffatom, an das sie angefügt sind, einen Pyrrolidin- oder Piperidinring bilden und $R^3$ und $R^4$ zusammen mit dem Stickstoffatom, an das sie angefügt sind, einen Piperidinring bilden oder jeweils für eine Methylgruppe stehen. Bei diesen besonders bevorzugten Verbindungen steht Q für einen Benzolring, dessen Einfügung in den Rest des Moleküls durch Bindungen in 1- und 3-Position erfolgt ist, während A für die Gruppierung $-O-(CH_2)_3-$ oder $-O-CH_2-CHOH-CH_2-$ steht. B bedeutet in diesem Fall die Gruppierung $-CH_2-S-CH_2CH_2-$, und Q' steht für einen in 2,5-Stellung in den Rest des Moleküls eingefügten Furanring oder einen in 2,5- bzw. 2,4-Stellung in den Rest des Moleküls eingefügten Thiophenring. R ist eines der oben genannten Ringsysteme.

Eine weitere, ganz besonders bevorzugte Gruppe von erfindungsgemäßen Verbindungen ist dadurch charakterisiert, daß in der allgemeinen Formel I Q und Q' jeweils für einen in 2,5-Position eingefügten Furanring oder einen in 2,5- oder 2,4-Position in den Rest des Moleküls eingefügten Thiophenring stehen. In diesem Falle haben sowohl m als auch n den Wert 1, und $R^1$, $R^2$, $R^3$ und $R^4$ haben die obigen Definitionen. A steht in diesem Fall weiterhin vorzugsweise für die Gruppierung $-CH_2-S-CH_2CH_2-$ oder $-CH_2-S-CH_2-CHOH-CH_2-$, während R wieder eines der oben genannten Ringsysteme bedeutet.

Eine Gruppe von Verbindungen, welche insbesondere in Betracht gezogen wird, ist dadurch charakterisiert, daß m und n jeweils den Wert 1 haben, $R^1$, $R^2$, $R^3$ und $R^4$ unabhängig voneinander jeweils eine Methyl- oder Ethylgruppe bedeuten, Q und Q' unabhängig voneinander jeweils für einen in 2,5-Position in den Rest des Moleküls eingefügten Furanring oder einen in 2,5- oder 2,4-Position eingefügten Thiophenring stehen, A und B, die in diesem Falle gleich sind, jeweils für die Gruppierung $-CH_2-S-CH_2CH_2-$ stehen und R eines der oben genannten Ringsysteme darstellt.

Ganz besonders bevorzugt werden Verbindungen, bei denen in der allgemeinen Formel I Q für einen in 1,3-Position in den Rest des Moleküls

eingefügten Benzolring steht und Q' für einen in 2,4-Position eingefügten Thiazolring steht. In diesem Falle haben m den Wert 1 und n den Wert 0, während $R^1$ und $R^2$ unabhängig voneinander einen Pyrrolidinring oder Piperidinring bedeuten und $R^3$ und $R^4$ miteinander für die Gruppierung $(NH_2)_2C=$ stehen, während A, B und R die oben genannten Bedeutungen besitzen.

Besonders bevorzugt werden folgende Verbindungen:

1-N-[3-(3-Piperidylmethyl)-phenoxy-2-hydroxy-propyl]-2-N'-[3-(3-piperidylmethyl)-phenoxy-propyl]-diaminocyclobuten-3,4-dion,

1,2-Bis-N,N'-[3-(3-piperidylmethyl)-phenoxy-2-hydroxypropyl]-diaminocyclobuten-3,4-dion und

3,4-Bis-[2-hydroxy-3-[3-(1-piperidylmethyl)-phenoxy]-propyl]-amino-1,2,5-thiadiazol-1-oxid.

Die erfindungsgemäßen Verbindungen können durch ein Verfahren hergestellt werden, das dadurch gekennzeichnet ist, daß man

- zur Herstellung von symmetrischen Verbindungen ein Derivat der allgemeinen Formel II

$$R^1R^2N-(CH_2)_m-Q-A-NH-Z \qquad (II)$$

das in an sich bekannter Weise - in situ - hergestellt werden kann, in der $R^1$, $R^2$, m, Q und A die in Anspruch 1 angegebene Bedeutung besitzen und Z für ein Ringsystem

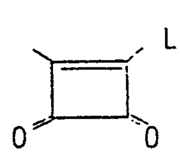 oder 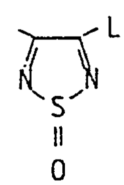

in dem L für eine Methoxy-, Ethoxy- oder Butoxygruppe als Austrittsgruppe steht, mit vorzugsweise äquimolaren Mengen eines Amins der allgemeinen Formel III

$$R^3R^4N-(CH_2)_n-Q'-B-NH_2 \qquad (III)$$

in der $R^3$, $R^4$, n, Q' und B wie oben definiert sind, in einem alkoholischen Lösungsmittel, wie Methanol, Ethanol, Isopropanol oder Butanol, vorzugsweise Ethanol, umsetzt und die erhaltene Verbindung gegebenenfalls in ihr physiologisch annehmbares Salz umwandelt.

Die Umsetzung erfolgt in üblicher Weise bei Raumtemperatur bis Siedetemperatur des verwendeten Lösungsmittels. Auch die Aufarbeitung und Isolierung erfolgt auf die übliche Art, beispielsweise durch Kristallisation etc.

Zur Herstellung von unsymmetrischen Verbindungen kann man ein Derivat der allgemeinen Formel II, wie oben beschrieben, mit einem Amin der allgemeinen Formel III, bei dem $R^3$, $R^4$, n, Q' und B nicht die gleiche Bedeutung besitzen wie $R^1$, $R^2$, m, Q und A, zu der entsprechenden erfindungsgemäßen Verbindung umsetzen.

Die Erfindung umfaßt auch die stereochemisch isomeren Verbindungen der Formel I in Form von physiologisch annehmbaren Hydraten und Salzen mit anorganischen und organischen Säuren. Diese Salze können zum Beispiel mit Mineralsäuren, wie Chlor-, Brom- und Jodwasserstoffsäure, Phosphorsäure, Metaphosphorsäure, Salpetersäure oder Schwefelsäure, oder mit organischen Säuren, wie Ameisensäure, Essigsäure, Propionsäure, Phenylessigsäure, Weinsäure, Zitronensäure, Fumarsäure, Methansulfonsäure etc., gebildet werden.

Die erfindungsgemäßen Verbindungen können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmazeutisch annehmbaren Trägern oder Verdünnungsmitteln hergestellt werden.

Die erfindungsgemäßen Verbindungen können daher für die orale, bukkale, topische, parenterale oder rektale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten. Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis insgesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in anderen Fällen die genannte obere Grenze überschritten werden muß.

Die erfindungsgemäßen Verbindungen zeichnen sich gegenüber anerkannt guten Arzneimitteln der gleichen Wirkungsrichtung durch eine Verbesserung der pharmakologischen Aktivitäten aus. Dies ergibt sich aus den Ergebnissen der im folgenden dargestellten pharmakologischen Vergleichsuntersuchungen.

Eine anerkannte Methode zur Bestimmung $H_2$-antagonistischer Wirksamkeit ist die Ermittlung der $pA_2$-Werte in vitro am isolierten Meerschweinchenvorhof (vgl. Ariens, Molecular Pharmacology, Band 1, Academic Press, New York, 1964)

| | $pA_2$-Werte | |
|---|---|---|
| Cimetidin | 6.21 | Vergleich |
| Beispiel 4 | 8.14 | |

Andere Verbindungen der allgemeinen Formel I zeigen ähnliche, pharmakologische Wirkungen.

Herstellungsbeispiel

1) Herstellung von

2-[2-Hydroxy-3-[3-(1-piperidylmethyl)benzyloxy]propyl]-1H-isoindol-1,3-dion

Eine Mischung aus 10,25 g (0,05 mol) 3-(1-Piperidylmethyl)-benzylalkohol und 10,15 g (0,05 mol) N-(2,3-Epoxypropyl)phthalimid wird 80 Minuten bei 130°C unter Stickstoff gerührt. Das erhaltene zähe Harz wird mit Methylenchlorid/Methanol 9 : 1 an Kieselgel chromatographiert. Die Hauptfraktion ergibt nach Eindampfen 8,60 g (42%) der Titelverbindung als hellbraunes Öl.

2) Herstellung von

2-Hydroxy-3-[3-(1-piperidylmethyl)benzyloxy]propylamin

8,60 g (0,021 mol) 2-[2-Hydroxy-3-[3-(1-piperidylmethyl)benzyloxy]propyl]-1H-isoindol-1,3-dion und 3,3 ml Hydrazinhydrat (80%) werden in 80 ml Ethanol 3 Stunden gekocht. Der nach Eindampfen der Mischung verbleibende Rückstand wird in 50 ml Wasser aufgenommen, mit 8 ml konz. Salzsäure versetzt und filtriert. Das Filtrat wird mit konz. Natronlauge auf pH 12 eingestellt und mit 3 x 40 ml Methylenchlorid extrahiert. Die organische Phase wird mit $Na_2SO_4$ getrocknet und im Vakuum eingedampft. Der verbleibende Rückstand wird im Hochvakuum destilliert. Man erhält 4,44 g (76%) eines farblosen Öls vom Sdp. 145 - 155° / $1x10^{-2}$ mbar.

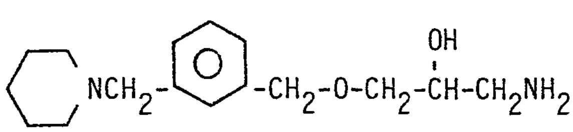

3) Herstellung von
1-Chlor-3-dibenzylamino-2-propanol

49,3 g (0,25 mol) Dibenzylamin und 25,4 g (0,275 mol) Epichlorhydrin werden 3 Stunden unter Stickstoff bei 85 - 90°C gerührt. Das erhaltene goldgelbe Öl wird im Hochvakuum destilliert. Man erhält 50,5 g (70%) eines farblosen Öls.

Sdp. 150 - 155°C (1,5 x $10^{-2}$ mbar)

4) Herstellung von
2-Dibenzylaminomethyl-oxiran

50,7 g (0,175 mol) 1-Chlor-3-dibenzylamino-2-propanol werden mit 9,5 g (0,24 Mol) Natriumhydroxid und 5 ml Wasser 1 Stunde bei 95°C gerührt. Nach Zugabe von 50 ml Chloroform und 20 ml Wasser werden die Phasen getrennt, die organische Phase wird mit 20 ml Wasser gewaschen, mit Natriumsulfat getrocknet und eingedampft. Die Destillation des erhaltenen gelben Öls im Vakuum ergibt 33,7 g (76%) des Oxirans als farbloses Öl.

Sdp. 125°C (1,5 x $10^{-2}$ mbar)

5) Herstellung von
1-Dibenzylamino-3-[3-(1-piperidylmethyl)phenyl]-2-propanol

Zu 0,97 g (0,04 mol) Magnesium-Spänen in 5 ml Tetrahydrofuran werden bei 60°C Innentemperatur 10,2 g (0,04 mol) 3-(1-Piperidylmethyl)-brombenzol in 20 ml Tetrahydrofuran getropft. Nach beendeter Zugabe wird 30 Minuten bei 60°C gerührt, dann wird die Lösung auf 10°C gekühlt. 10,2 g (0,04 mol) 2-Dibenzylaminomethyl-oxiran in 20 ml Tetrahydrofuran werden langsam zugetropft, und die erhaltene Lösung wird 2 Stunden bei Raumtemperatur gerührt. Nach Zugabe von 20 ml Eiswasser und 4,5 g Ammoniumchlorid wird die wäßrige Phase abgetrennt und mit 25 ml Methylenchlorid extrahiert. Die

vereinigten organischen Phasen ergeben nach Trocknen mit Natriumsulfat und Einengen ein gelbes Harz, das mit Methylenchlorid/Methanol (9:1) an Kieselgel chromatographiert wird. Die 2. Fraktion liefert nach Einengen die Titelverbindung als schwachgelbes Öl.

Ausbeute: 11,5 g (67%)

6) Herstellung von
1-Amino-3-[3-(1-piperidylmethyl)phenyl]-2-propanol

11,5 g (0,027 mol) 1-Dibenzylamino-3-[3-(1-piperidylmethyl)phenyl]-2-propanol in 90 ml Ethanol und 10 ml Wasser werden in Gegenwart von 0,5 g Palladium/Aktivkohle (10% Pd) bei 35°C und Atmosphärendruck hydriert. Nach Abfiltrieren des Katalysators und Abdampfen des Lösungsmittels verbleiben 5,8 g eines farblosen Öls, das nach chromatographischer Reinigung mit Methanol/Ammoniak konz. (95:5) 3,5 g (52%) der Titelverbindung in Form eines farblosen Öls ergibt.

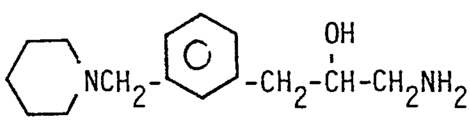

7) Herstellung von
2-Hydroxy-3-[3-(1-piperidylmethyl)phenyl]-butyronitril

0,74 g (3,2 mmol) 2-[3-(1-Piperidylmethyl)phenyl]methyl-oxiran, 0,294 g (6 mmol) Natriumcyanid und 0,107 g (2 mmol) Ammoniumchlorid werden in 5 ml Ethanol/5 ml Wasser 6 Stunden gekocht. Nach weitgehendem Einengen der Mutterlauge im Vakuum wird der Rückstand in 10 ml Wasser aufgenommen, die Lösung mit Kaliumcarbonat auf pH 12 eingestellt und mit 3 x 20 ml Methylenchlorid extrahiert. Die organische Phase ergibt nach Trocknen und Eindampfen im Vakuum 0,82 g eines braunen Öls.

8) Herstellung von
4-Amino-1-[3-(1-piperidylmethyl)phenyl]-2-butanol

Zu 0,82 g (3,2 mmol) 2-Hydroxy-3-[3-(1-piperidylmethyl)phenyl]-butyronitril in 30 ml Ether und 10 ml Tetrahydrofuran werden 0,10 g (2,6 mmol) Lithiumaluminiumhydrid gegeben und die Mischung 2 Stunden zum Rückfluß erhitzt. Nach Zugabe von 0,25 ml Wasser wird der entstandene Niederschlag abgesaugt, mit 20 ml Methylenchlorid aufgeschlämmt und nochmals abgesaugt. Nach Trocknen der vereinigten organischen Phasen und Eindampfen im Vakuum verbleiben 0,65 g eines gelben Öls, die im Hochvakuum destilliert 0,51 g (61%) der Titelverbindung ergeben.

Farbloses, zähes Öl, Sdp. 140 - 150°C/7 x $10^{-3}$ mbar.

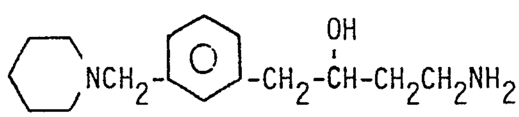

9) Herstellung von
2-[2-Hydroxy-3-[5-(1-piperidylmethyl)-2-thienylthio]propyl]-1H-isoindol-1,3-dion

Eine Mischung aus 6,84 g (20 mmol) 5-(1-Piperidylmethyl)-2-S-isothioharnstoffmethylthiophendihydrochlorid und 6,1 g (30 mmol) N-(2,3-Epoxypropyl)-phthalimid wird in 50 ml Ethanol vorgelegt und bei 0 - 5°C langsam mit 2,4 g (60 mmol) NaOH, gelöst in 60 ml Ethanol, versetzt. Anschließend läßt man 1 h bei 0 - 5°C, dann 3 h bei Raumtemperatur reagieren. Die Reaktionslösung wird im Vakuum eingeengt, der Rückstand in $CH_2Cl_2$/MeOH (80 : 20) aufgenommen, die organische Phase mit Wasser neutralgewaschen, über $Na_2SO_4$ getrocknet und im Vakuum eingeengt. Man erhält 8,3 g (96%) der Titelverbindung als braunes Öl.

0158789

10) Herstellung von

2-Hydroxy-3-[5-(1-piperidylmethyl)-2-thienylthio]propylamin

8,60 g (20 mmol) 2-[2-Hydroxy-3-[5-(1-piperidylmethyl)-2-thienylthio]-propyl]-1H-isoindol-1,3-dion und 3,3 ml Hydrazinhydrat (80%) werden in 80 ml Ethanol 3 Stunden gekocht. Der nach Einengen der Mischung verbleibende Rückstand wird in 50 ml Wasser aufgenommen, mit 8 ml konz. Salzsäure versetzt und filtriert. Das Filtrat wird mit konz. Natronlauge auf pH 12 eingestellt und mit 3 x 40 ml Methylenchlorid extrahiert. Die organische Phase wird mit $Na_2SO_4$ getrocknet und im Vakuum eingedampft. Man erhält 4,9 g (82% d.Th.) der Titelverbindung als hellgrünes Öl.

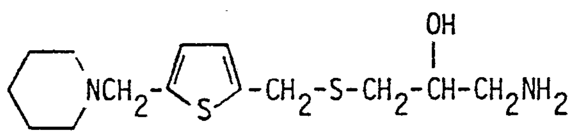

0158789

Beispiel 1

Herstellung von 1-N-[3-(3-Piperidylmethyl)-phenoxy-2-hydroxy-propyl]-2-N'-[3-(3-piperidylmethyl)-phenoxy-propyl]-diaminocyclobuten-3,4-dion

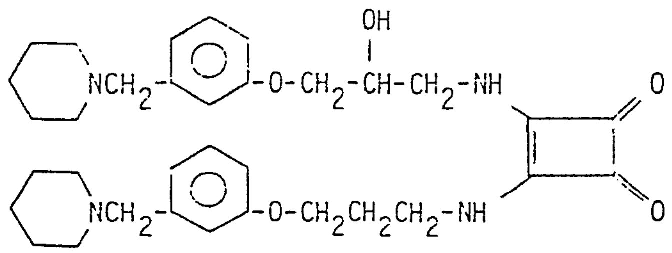

6.78 g (30 mmol) Quadratsäuredibutylester werden in 45 ml absolutem Ethanol mit 7.44 g (30 mmol) (3-Piperidylmethyl-phenoxy)-propylamin bei Raumtemperatur 6 h umgesetzt. Anschließend gibt man 7.92 g (30 mmol) (3-Piperidylmethyl-phenoxy)-2-hydroxypropylamin zu und läßt 4 h reagieren. Der anfallende Feststoff wird abgesaugt und aus Ethanol umkristallisiert.

Farblose Kristalle vom Schmelzpunkt 152 - 156 °C

Rf = 0.48 ($C_2H_5OH$ / $N(C_2H_5)_3$ 97 : 3)

Ausbeute: 12.8 g (72 % d.Th.)

$C_{34}H_{46}N_4O_5$ (590.8) Ber.: C 69.12 H 7.85 N 9.48

Gef.: C 69.21 H 7.74 N 9.77

$^1$H-NMR-Spektrum: (d$_6$-DMSO, TMS als interner Standard)

$\delta$ = 1.20 - 1.67 (m) 2 H,
2.00 (m) 2 H,
2.20 - 2.43 (m) 8 H,
3.37 (s) 4 H,
3.53 - 3.87 (m) 4 H,
3.93 - 4.20 (m) 5 H,
5.43 (s, breit) (austauschbar mit $D_2O$) 1 H,

6.70 - 7.33 (m) 8 H,
7.90 (m) (austauschbar mit $D_2O$) 2 H ppm.

Beispiel 2

Herstellung von 1,2-Bis-N,N'-[3-(3-piperidylmethyl)-phenoxy-propyl]-diamino-cyclobuten-3,4-dion

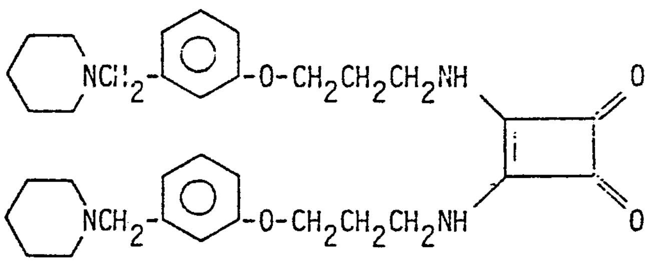

Die Herstellung erfolgt analog Beispiel 1 aus 6.87 g (30 mmol) Quadratsäuredibutylester und 14.88 g (60 mmol) (3-Piperidylmethyl-phenoxy)-propylamin.

Farblose Kristalle vom Schmelzpunkt 158 - 160 °C

Rf = 0.49 ($C_2H_5OH$ / $N(C_2H_5)_3$ 97 : 3)

Ausbeute: 12 g (70 % d.Th.)

$C_{34}H_{46}N_4O_4$ (574.8) Ber.: C 71.04 H 8.07 N 9.75
Gef.: C 70.81 H 7.81 N 9.79

$^1$H-NMR-Spektrum: ($CF_3$ COOD, TMS als interner Standard) $\delta$ = 1.40 - 2.50 (m) 16 H,
2.80 - 3.27 (m) 4 H,
3.50 - 3.83 (m) 4 H,
4.00 - 4.47 (m) 12 H,
6.93 - 7.60 (m) 8 H ppm.

0158789

Beispiel 3

Herstellung von 1,2-Bis-N,N'-[3-(3-piperidylmethyl)-phenoxy-2-hydroxypropyl]-diamino-cyclobuten-3,4-dion

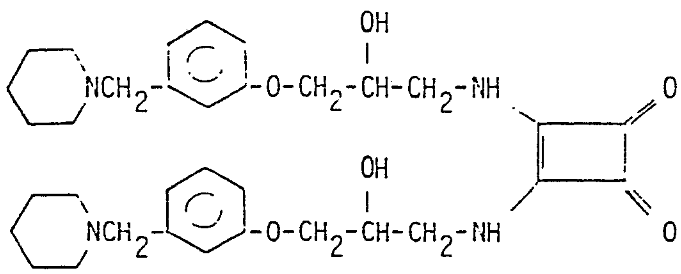

Die Herstellung erfolgt analog Beispiel 1 aus 6.87 g (30 mmol) Quadratsäuredibutylester und 15.84 g (60 mmol) (3-Piperidylmethyl-phenoxy)-2-hydroxy-propylamin

Farblose Kristalle vom Schmelzpunkt 108 - 110 °C

Rf = 0.46 ($C_2H_5OH$ / $N(C_2H_5)_3$ 97 : 3)

Ausbeute: 10.36 g (57 % d.Th.)

| | | | | |
|---|---|---|---|---|
| $C_{34}H_{46}N_4O_6$ (606.8) | Ber.: | C 67.30 | H 7.64 | N 9.23 |
| | Gef.: | C 67.27 | H 7.53 | N 9.00 |

$^1$H-NMR-Spektrum: (d$_6$-DMSO, TMS als interner Standard)

$\delta$ = 1.20 - 1.67 (m) 12 H,
2.17 - 2.47 (m) 8 H,
3.40 (s) 4 H,
3.50 - 4.13 (m) 10 H,
5.43 (s, breit) (austauschbar mit $D_2O$) 2 H,
6.70 - 7.33 (m) 8 H,
7.67 (t) (austauschbar mit $D_2O$) 2 H ppm.

0158789

Beispiel 4

Herstellung von 3,4-Bis[2-hydroxy-3-[3-(1-piperidylmethyl)phenoxy] propyl]-amino-1,2,5-thiadiazol-1-oxid

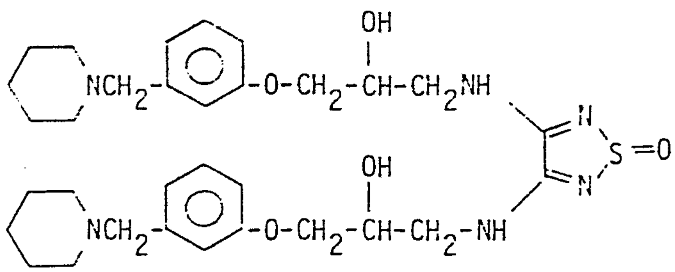

Die Herstellung erfolgt analog Beispiel 1 aus 9.0 g (30 mmol) 3,4-Diethoxy-1,2,5-thiadiazol-1-oxid und 15.84 g (60 mmol) (3-Piperidylmethyl-phenoxy) -2-hydroxy-propylamin.

Farblose Kristalle vom Schmelzpunkt 129 - 130 °C

Rf = 0.60 ($CH_3OH$ / $NH_3$ conc. 95 : 5)

Ausbeute: 6.9 g (37 % d.Th.)

$C_{32}H_{46}N_6O_5S$ (626.8)

$^1$H-NMR-Spektrum: (CDCl$_3$, TMS als interner Standard)

$\delta$ = 1.20 - 1.73 (m) 12 H,
2.17 - 2.47 (m) 8 H,
3.33 (s) 4 H,
3.50 - 3.77 (m) 4 H,
3.78 - 4.00 (m) 4 H,
4.27 (m) 2 H,
5.93 (m, breit) (austauschbar mit $D_2O$) 4 H,
6.53 - 7.20 (m) 8 H ppm.

0158789

Beispiel 5

Herstellung von 1,2-Bis-N,N'-[[3-[5-dimethylaminomethylfuranyl-2-methyl]-thio]ethylamin]-cyclobuten-3,4-dion

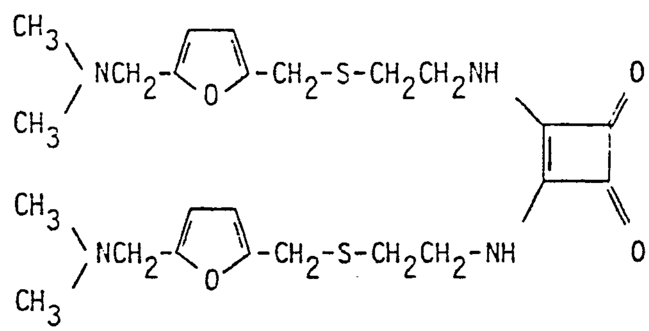

Die Herstellung erfolgt analog Beispiel 1 aus 6,87 g (30 mmol) Quadratsäuredibutylester und 12,84 g (60 mmol) [5-Dimethylaminomethylfuranyl-2-methyl]-thioethylamin.

Farblose Kristalle vom Schmelzpunkt 104 - 106 °C

Rf = 0.35 ($C_2H_5OH$ / $N(C_2H_5)_3$ 97 : 3)

Ausbeute: 7,13 g (47 % d.Th.)

$C_{24}H_{34}N_4O_4S_2$ (506)

$^1$H-NMR-Spektrum: (d$_6$-DMSO, TMS als interner Standard)

$\delta$ = 2.17 (s) 12 H,
2.67 (t) 4 H,
3.37 (s) 4 H,
3.67 (t) 4 H,
3.80 (s) 4 H,
6.23 (m) 4 H,
7.67 (t, breit) (austauschbar mit $D_2O$) 2 H ppm.

0158789

Beispiel 6

Herstellung von 1-N'-[3-(3-Piperidylmethyl)-phenoxy-propyl]-2-N'-[[3-[5-dimethylaminomethylfuranyl-2-methyl]-thio]-ethyl]-diaminocyclobuten-3,4-dion

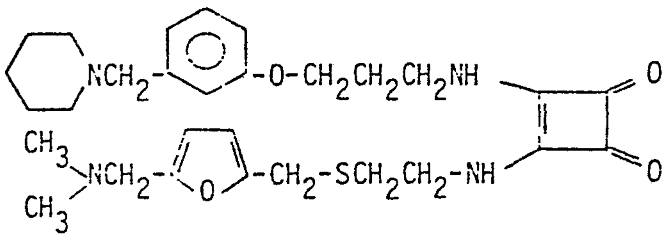

Die Herstellung erfolgt analog Beispiel 1 aus 6,87 g (30 mmol) Quadratsäuredibutylester, 7,44 g (30 mmol) (3-Piperidylmethyl-phenoxy)-propylamin und 6,42 g (30 mmol) [5-Dimethylaminomethylfuranyl-2-methyl]-thio-ethylamin.

Amorphe Festsubstanz

Rf = 0.40 ($C_2H_5OH$ / $N(C_2H_5)_3$ 97 : 3)

Ausbeute: 9,07 g (56 % d.Th.)

$C_{29}H_{40}N_4O_4S$ (540)

| | |
|---|---|
| $^1$H-NMR-Spektrum: | $\delta$ = 1.45 (m) 6 H, |
| ($d_6$-DMSO, TMS als | 2.00 (t) 2 H, |
| interner Standard) | 2.13 (s) 6 H, |
| | 2.30 (m) 4 H, |
| | 2.67 (t) 4 H, |
| | 3.37 (s) 2 H, |
| | 3.50 - 3.87 (m) 6 H, |
| | 4.00 (t) 2 H, |
| | 6.17 (m) 2 H, |
| | 6.67 - 7.33 (m) 4 H, |
| | 7.60 (breit) (austauschbar mit $D_2O$) 2 H ppm. |

PATENTANSPRÜCHE

1. Diaminderivate der allgemeinen Formel I

$$R^1R^2N\text{-}(CH_2)_m\text{-}Q\text{-}A\text{-}NH\text{-}R\text{-}NH\text{-}B\text{-}Q'\text{-}(CH_2)_nNR^3R^4 \qquad (I)$$

in der m und n unabhängig voneinander den Wert 0 oder 1 haben, $R^1$, $R^2$, $R^3$ und $R^4$, die gleich oder verschieden sein können, jeweils für Wasserstoff, $C_{1-10}$-Alkyl, $C_{5-6}$-Cycloalkyl, Amino, Niedrigalkylamino oder Diniedrigalkylamino stehen oder $R^1$ und $R^2$ und/oder $R^3$ und $R^4$ zusammen mit dem Stickstoffatom, an das sie jeweils gebunden sind, einen 5- bis 8gliedrigen alicyclischen, substituierten oder unsubstituierten heterocyclischen Ring bilden oder $R^3$ und $R^4$ miteinander die Gruppierung $(NH_2)_2C=$ bilden oder $R^1$ und $R^2$ im Fall, daß m den Wert 0 hat, miteinander die Gruppierung $(NH_2)_2C=$ bilden, Q und Q' unabhängig voneinander einen Furan-, Thiophen-, Thiazol- oder Benzolring bedeuten, A und B unabhängig voneinander für eine Gruppierung $-O-(CH_2)_q-$, $-O-CH_2-CHOH-CH_2-$ oder $-CH_2-X-CH_2-Y-(CH_2)_p-$ stehen, q den Wert 2, 3 oder 4 hat, X ein

Schwefel- oder Sauerstoffatom oder die Gruppe -CHOH ist, Y eine Einfachbindung oder die Gruppe -CHOH ist, p den Wert 1 oder 2 hat und R für das Ringsystem

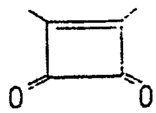

oder

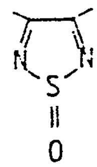

steht, sowie die physiologisch annehmbaren Salze und Hydrate davon.

2. Diaminderivate nach Anspruch 1, dadurch gekennzeichnet, daß m und n jeweils den Wert 1 haben, $R^1$, $R^2$, $R^3$ und $R^4$, die gleich oder verschieden sein können, jeweils für $C_{1-3}$-Alkyl oder $C_{5-6}$-Cycloalkyl stehen oder $R^1$ und $R^2$ und/oder $R^3$ und $R^4$ zusammen mit dem Stickstoffatom, an das sie jeweils gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen Benzolring, der in 1,3- oder 1,4-Position in den Rest des Moleküls eingefügt ist, steht, A die Gruppierung $-O-(CH_2)_q-$, $-O-CH_2-CHOH-CH_2-$ oder $-CH_2-O-CH_2-CHOH-CH_2-$ bedeutet, R für

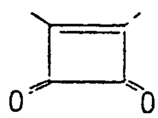

oder

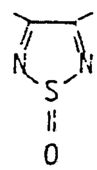

steht, B für die Gruppierung $-O-(CH_2)_q-$, $-O-CH_2-CHOH-CH_2-$ oder $-CH_2-O-CH_2-CHOH-CH_2-$ steht und Q' einen Benzolring, der in 1,3- oder 1,4-Position in den Rest des Moleküls eingefügt ist, bedeutet.

3. Diaminderivate nach Anspruch 1, dadurch gekennzeichnet, daß m und n jeweils den Wert 1 haben, $R^1$, $R^2$, $R^3$ und $R^4$, die gleich oder verschieden sein können, jeweils für $C_{1-3}$-Alkyl oder $C_{5-6}$-Cycloalkyl stehen oder $R^1$ und $R^2$ und/oder $R^3$ und $R^4$ zusammen mit dem Stickstoffatom, an das sie jeweils gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen Benzolring, der in 1,3- oder 1,4-Position in den Rest des Moleküls eingearbeitet ist, steht, A die Gruppierung $-O-(CH_2)_q-$, $-O-CH_2-CHOH-CH_2-$ oder $-CH_2-O-CH_2-CHOH-CH_2-$ bedeutet, R die in Anspruch 1 angegebene Bedeutung hat, B für die Gruppierung $-CH_2-CHOH-CH_2-$ oder $-CH_2-CHOH-CH_2CH_2-$ steht und Q' die gleiche Bedeutung wie Q besitzt.

4. Diaminderivate nach Anspruch 1, dadurch gekennzeichnet, daß m und n jeweils den Wert 1 haben, $R^1$, $R^2$, $R^3$ und $R^4$ die in Anspruch 1 angegebenen Bedeutungen besitzen, Q für einen in 1,3- oder 1,4-Position in den Rest des Moleküls eingefügten Benzolring steht, A die Gruppierung $-O-(CH_2)_q-$, $-O-CH_2-CHOH-CH_2-$, $-CH_2-O-CH_2-CHOH-CH_2$, $-CH_2-CHOH-CH_2-$ oder $-CH_2-CHOH-CH_2CH_2-$ bedeutet, R die in Anspruch 1 angegebene Bedeutung besitzt, B für die Gruppierung $-CH_2-S-CH_2CH_2-$ oder $-CH_2-S-CH_2-CHOH-CH_2-$ steht und Q' einen Furan- oder Thiophenring bedeutet, wobei der Furanring in 2,5-Position und der Thiophenring in 2,4- oder 2,5-Position in den Rest des Moleküls eingefügt sind.

5. Diaminderivate nach Anspruch 1, dadurch gekennzeichnet, daß m und n jeweils den Wert 1 haben, $R^1$, $R^2$, $R^3$ und $R^4$ die in Anspruch 1 angegebenen Bedeutungen besitzen, Q für einen in 2,5-Position in den Rest des Moleküls eingefügten Furanring oder einen in 2,4- oder 2,5-Position in den Rest des Moleküls eingefügten Thiophenring steht, A die Gruppierung $-CH_2-S-CH_2CH_2-$ oder $-CH_2-S-CH_2-CHOH-CH_2-$ bedeutet, R die in Anspruch 1 angegebene Bedeutung besitzt, B für die Gruppierung $-CH_2-S-CH_2CH_2-$ oder $-CH_2-S-CH_2-CHOH-CH_2-$ steht und Q' einen Furan- oder Thiophenring bedeutet, wobei der Furanring in 2,5-Position und der Thiophenring in 2,4- oder 2,5-Position in den Rest des Moleküls eingefügt sind.

6. Diaminderivate nach Anspruch 1, dadurch gekennzeichnet, daß m den Wert 0 hat, $R^1$ und $R^2$ miteinander die Gruppierung $(NH_2)_2C=$ bilden und $R^3$ und $R^4$ die in Anspruch 1 angegebenen Bedeutungen besitzen, Q für einen in 2,4-Position in den Rest des Moleküls eingefügten Thiazolring steht, A die Gruppierung $-CH_2-S-CH_2CH_2-$ bedeutet, R

und B die in Anspruch 1 angegebenen Bedeutungen besitzen und Q' einen in 1,3- oder 1,4-Position in den Rest des Moleküls eingefügten Benzolring, einen in 2,5-Position in den Rest des Moleküls eingefügten Furanring oder einen in 2,5- oder 2,4-Position in den Rest des Moleküls eingefügten Thiophenring bedeutet.

7. 1-N-[3-(3-Piperidylmethyl)-phenoxy-2-hydroxy-propyl]-2-N'-[3-(3-piperidylmethyl)-phenoxy-propyl]-diaminocyclobuten-3,4-dion sowie die physiologisch annehmbaren Salze und Hydrate davon.

8. 1,2-Bis-N,N'-[3-(3-piperidylmethyl)-phenoxy-2-hydroxypropyl]-diamino-cyclobuten-3,4-dion sowie die physiologisch annehmbaren Salze und Hydrate davon.

9. 3,4-Bis-[2-hydroxy-3-[3-(1-piperidylmethyl)-phenoxy]-propyl]-amino-1,2,5-thiadiazol-1-oxid sowie die physiologisch annehmbaren Salze und Hydrate davon.

10. Verfahren zur Herstellung der Diaminderivate nach den Ansprüchen 1 bis 9, dadurch g e k e n n z e i c h n e t , daß man eine Verbindung der allgemeinen Formel II

$$R^1R^2N-(CH_2)_m-Q-A-NH-Z \qquad (II)$$

in der $R^1$, $R^2$, m, Q und A die in Anspruch 1 angegebenen Bedeutungen haben und Z für den Rest

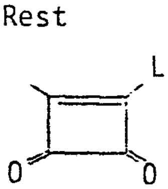

oder

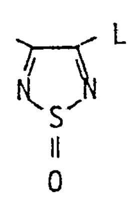

steht, wobei L eine Methoxy-, Ethoxy- oder Butoxygruppe als Austrittsgruppe bedeutet, mit einem Amin der allgemeinen Formel III

$$R^3R^4N-(CH_2)_n-Q'-B-NH_2 \qquad (III)$$

worin $R^3$, $R^4$, n, Q' und B die in Anspruch 1 angegebenen Bedeutungen haben, in an sich bekannter Weise zu einer Verbindung der allgemeinen Formel I umsetzt und diese gegebenenfalls in ihr physiologisch annehmbares Salz umwandelt.

11. Arzneimittel, dadurch gekennzeichnet, daß es zusammen mit einem inerten, pharmazeutisch annehmbaren Träger oder Verdünnungsmittel eine Verbindung nach einem der Ansprüche 1 bis 9 enthält.

# P A T E N T A N S P R O C H E

## für den Vertragsstaat AT

1. Verfahren zur Herstellung von Diaminderivaten der allgemeinen Formel I

$$R^1R^2N-(CH_2)_m-Q-A-NH-R-NH-B-Q'-(CH_2)_nNR^3R^4 \qquad (I)$$

in der m und n unabhängig voneinander den Wert 0 oder 1 haben, $R^1$, $R^2$, $R^3$ und $R^4$, die gleich oder verschieden sein können, jeweils für Wasserstoff, $C_{1-10}$-Alkyl, $C_{5-6}$-Cycloalkyl, Amino, Niedrigalkylamino oder Diniedrigalkylamino stehen oder $R^1$ und $R^2$ und/oder $R^3$ und $R^4$ zusammen mit dem Stickstoffatom, an das sie jeweils gebunden sind, einen 5- bis 8gliedrigen alicyclischen, substituierten oder unsubstituierten heterocyclischen Ring bilden oder $R^3$ und $R^4$ miteinander die Gruppierung $(NH_2)_2C=$ bilden oder $R^1$ und $R^2$ im Fall, daß m den Wert 0 hat, miteinander die Gruppierung $(NH_2)_2C=$ bilden, Q und Q' unabhängig voneinander einen Furan-, Thiophen-, Thiazol- oder Benzolring bedeuten, A und B unabhängig voneinander für eine Gruppierung $-O-(CH_2)_q-$, $-O-CH_2-CHOH-CH_2-$ oder $-CH_2-X-CH_2-Y-(CH_2)_p-$ stehen, q den Wert 2, 3 oder 4 hat, X ein Schwefel- oder Sauerstoffatom oder die Gruppe -CHOH ist, Y eine Einfachbindung oder die Gruppe -CHOH ist, p den Wert 1 oder 2 hat und R für das Ringsystem

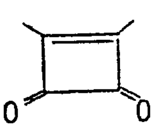

oder

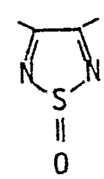

steht, sowie die physiologisch annehmbaren Salze und Hydrate davon, dadurch g e k e n n z e i c h n e t , daß man eine Verbindung der allgemeinen Formel II

$$R^1R^2N-(CH_2)_m-Q-A-NH-Z \qquad (II)$$

in der $R^1$, $R^2$, m, Q und A die oben angegebenen Bedeutungen haben und Z für den Rest

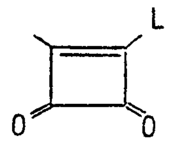 oder 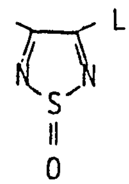

steht, wobei L eine Methoxy-, Ethoxy- oder Butoxygruppe als Austrittsgruppe bedeutet, mit einem Amin der allgemeinen Formel III

$$R^3R^4N-(CH_2)_n-Q'-B-NH_2 \qquad (III)$$

worin $R^3$, $R^4$, n, Q' und B die oben angegebenen Bedeutungen haben, in an sich bekannter Weise zu einer Verbindung der allgemeinen Formel I umsetzt und diese gegebenenfalls in ihr physiologisch annehmbares Salz umwandelt.

2. Verfahren nach Anspruch 1 zur Herstellung von Verbindungen, bei denen m und n jeweils den Wert 1 haben, $R^1$, $R^2$, $R^3$ und $R^4$, die gleich oder verschieden sein können, jeweils für $C_{1-3}$-Alkyl oder $C_{5-6}$-Cycloalkyl stehen oder $R^1$ und $R^2$ und/oder $R^3$ und $R^4$ zusammen mit dem Stickstoffatom, an das sie jeweils gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen Benzolring, der in 1,3- oder 1,4-Position in den Rest des Moleküls eingefügt ist, steht, A die Gruppierung $-O-(CH_2)_q-$, $-O-CH_2-CHOH-CH_2-$ oder $-CH_2-O-CH_2-CHOH-CH_2-$ bedeutet, R für

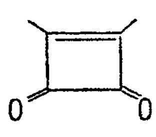 oder 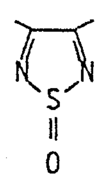

steht, B für die Gruppierung $-O-(CH_2)_q-$, $-O-CH_2-CHOH-CH_2-$ oder $-CH_2-O-CH_2-CHOH-CH_2-$ steht und Q' einen Benzolring, der in 1,3- oder 1,4-Position in den Rest des Moleküls eingefügt ist, bedeutet.

3. Verfahren nach Anspruch 1 zur Herstellung von Verbindungen, bei denen m und n jeweils den Wert 1 haben, $R^1$, $R^2$, $R^3$ und $R^4$, die

gleich oder verschieden sein können, jeweils für $C_{1-3}$-Alkyl oder $C_{5-6}$-Cycloalkyl stehen oder $R^1$ und $R^2$ und/oder $R^3$ und $R^4$ zusammen mit dem Stickstoffatom, an das sie jeweils gebunden sind, einen Pyrrolidin-, Methylpyrrolidin-, Morpholin-, Thiomorpholin-, Piperidin-, Methylpiperidin-, N-Methylpiperazin-, Homopiperidin-, Heptamethylenimino- oder Octamethyleniminoring bilden, Q für einen Benzolring, der in 1,3- oder 1,4-Position in den Rest des Moleküls eingearbeitet ist, steht, A die Gruppierung $-O-(CH_2)_q-$, $-O-CH_2-CHOH-CH_2-$ oder $-CH_2-O-CH_2-CHOH-CH_2-$ bedeutet, R die in Anspruch 1 angegebene Bedeutung hat, B für die Gruppierung $-CH_2-CHOH-CH_2-$ oder $-CH_2-CHOH-CH_2CH_2-$ steht und Q' die gleiche Bedeutung wie Q besitzt.

4. Verfahren nach Anspruch 1 zur Herstellung von Verbindungen, bei denen m und n jeweils den Wert 1 haben, $R^1$, $R^2$, $R^3$ und $R^4$ die in Anspruch 1 angegebenen Bedeutungen besitzen, Q für einen in 1,3- oder 1,4-Position in den Rest des Moleküls eingefügten Benzolring steht, A die Gruppierung $-O-(CH_2)_q-$, $-O-CH_2-CHOH-CH_2-$, $-CH_2-O-CH_2-CHOH-CH_2$, $-CH_2-CHOH-CH_2-$ oder $-CH_2-CHOH-CH_2CH_2-$ bedeutet, R die in Anspruch 1 angegebene Bedeutung besitzt, B für die Gruppierung $-CH_2-S-CH_2CH_2-$ oder $-CH_2-S-CH_2-CHOH-CH_2-$ steht und Q' einen Furan- oder Thiophenring bedeutet, wobei der Furanring in 2,5-Position und der Thiophenring in 2,4- oder 2,5-Position in den Rest des Moleküls eingefügt sind.

5. Verfahren nach Anspruch 1 zur Herstellung von Verbindungen, bei denen m und n jeweils den Wert 1 haben, $R^1$, $R^2$, $R^3$ und $R^4$ die in Anspruch 1 angegebenen Bedeutungen besitzen, Q für einen in 2,5-Position in den Rest des Moleküls eingefügten Furanring oder einen in 2,4- oder 2,5-Position in den Rest des Moleküls eingefügten Thiophenring steht, A die Gruppierung $-CH_2-S-CH_2CH_2-$ oder $-CH_2-S-CH_2-CHOH-CH_2-$ bedeutet, R die in Anspruch 1 angegebene Bedeutung besitzt, B für die Gruppierung $-CH_2-S-CH_2CH_2-$ oder $-CH_2-S-CH_2-CHOH-CH_2-$ steht und Q' einen Furan- oder Thiophenring bedeutet, wobei der Furanring in 2,5-Position und der Thiophenring in 2,4- oder 2,5-Position in den Rest des Moleküls eingefügt sind.

6. Verfahren nach Anspruch 1 zur Herstellung von Verbindungen, bei denen m den Wert 0 hat, $R^1$ und $R^2$ miteinander die Gruppierung $(NH_2)_2C=$ bilden und $R^3$ und $R^4$ die in Anspruch 1 angegebenen Bedeutungen besitzen, Q für einen in 2,4-Position in den Rest des Moleküls eingefügten Thiazolring steht, A die Gruppierung $-CH_2-S-CH_2CH_2-$ bedeutet, R und B die in Anspruch 1 angegebenen Bedeutungen besitzen und Q' einen in 1,3- oder 1,4-Position in den Rest des Moleküls eingefügten Benzolring, einen in 2,5-Position in den Rest des Moleküls eingefügten Furanring oder einen in 2,5- oder 2,4-Position in den Rest des Moleküls eingefügten Thiophenring bedeutet.

7. Verfahren nach Anspruch 1 zur Herstellung von 1-N-[3-(3-Piperidylmethyl)-phenoxy-2-hydroxy-propyl]-2-N'-[3-(3-piperidylmethyl)-phenoxy-propyl]-diaminocyclobuten-3,4-dion sowie der physiologisch annehmbaren Salze und Hydrate davon.

8. Verfahren nach Anspruch 1 zur Herstellung von 1,2-Bis-N,N'-[3-(3-piperidylmethyl)-phenoxy-2-hydroxypropyl]-diamino-cyclobuten-3,4-dion sowie der physiologisch annehmbaren Salze und Hydrate davon.

9. Verfahren nach Anspruch 1 zur Herstellung von 3,4-Bis-[2-hydroxy-3-[3-(1-piperidylmethyl)-phenoxy]-propyl]-amino-1,2,5-thiadiazol-1-oxid sowie der physiologisch annehmbaren Salze und Hydrate davon.

Europäisches Patentamt

# EUROPÄISCHER RECHERCHENBERICHT

0158789 Nummer der Anmeldung

EP 85101912.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US - A - 4 062 863 (GANELLIN et al.)<br>* Beispiele 2,5; Ansprüche 1,6 *<br>-- | 1,10, 11 | C 07 D 295/08<br>C 07 D 307/52<br>C 07 D 285/10<br>A 61 K 31/445<br>A 61 K 31/425<br>A 61 K 31/34 |
| A | GB - A - 2 098 988 (BRISTOL-MYERS)<br>* Ansprüche 1,42,48 *<br>-- | 1,10, 11 | |
| A | DE - A1 - 3 324 771 (GLAXO GROUP LTD.)<br>* Ansprüche 1,8,9 *<br>-- | 1,10, 11 | |
| A | CHEMICAL ABSTRACTS, Band 96, Nr. 10, 8. März 1982, Columbus, Ohio, USA<br>A.A. ALGIERE et al. "1,2,5-Thiadiazole 1-oxide and 1,1-dioxide derivatives. A new class of potent histamine $H_2$-receptor antagonists"<br>Seite 23, Zusammenfassung-Nr. 79 442j<br>& J. Med. Chem. 1982, 25(3), 210-12<br>-- | 1,11 | RECHERCHIERTE SACHGEBIETE (Int. Cl.4)<br>C 07 D 295/00<br>C 07 D 307/00<br>C 07 D 285/00 |

Der vorliegende Recherchenbericht wurde für alle Patentansprüche erstellt

| Recherchenort | Abschlußdatum der Recherche | Prüfer |
|---|---|---|
| WIEN | 22-05-1985 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82

Europäisches Patentamt

# EUROPÄISCHER RECHERCHENBERICHT

0158789

Nummer der Anmeldung



EP 85101912.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | CHEMICAL ABSTRACTS, Band 96, Nr. 10, 8. März 1982, Columbus, Ohio, USA<br>W.C. LUMMA et al. "Inhibitors of gastric acid secretion: 3,4-diamino-1,2,5-thiadiazole 1-oxides and 1,1-dioxides as urea equivalents in a series of histamine $H_2$receptor antagonists"<br>Seite 23, Zusammenfassung-Nr. 79 443k<br>& J. Med. Chem. 1982, 25(3), 207-10<br>---- | 1,11 | |
| | | | RECHERCHIERTE SACHGEBIETE (Int. Cl 4) |

Der vorliegende Recherchenbericht wurde für alle Patentansprüche erstellt

| Recherchenort | Abschlußdatum der Recherche | Prüfer |
|---|---|---|
| WIEN | 22-05-1985 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82